# EUROPEAN PATENT APPLICATION

(11) **EP 4 309 578 A1**
(43) Date of publication of application: **24.01.2024**
(21) Application number: 23153014.8
(22) Date of filing: 24.01.2023
(51) Int. Cl.: A61B 5/107

(54) **FIXTURE ASSEMBLY AND METHOD FOR POSITIONING APPENDAGE FOR DIGITAL SCANNING**

(30) Priority: 22.07.2022 US 202263369163 P; 17.08.2022 US 202217890231
(71) Applicant: Comb O&P, Chardon, OH 44024 (US)
(72) Inventor: Goodman, Alex, Solon, 44139 (US); Naft, Jonathan, Chagrin Falls, 44023 (US)
(74) Representative: Forresters IP LLP

(57) **Abstract**

Provided herein is an adjustable fixture assembly for supporting an appendage of a patient, such as a foot and/or leg, in a desirable position to allow for a comprehensive scan of the appendage. The adjustable fixture assembly comprising a base, a support member, and a platform. A ball joint secures a first end of the support member to the based and a second end of the support member is coupled to the platform. The adjustable fixture assembly is configured to support the foot and/or leg of a patient on the platform, which is configured to pivot about three degrees of freedom relative to the base. The adjustable fixture assembly can optionally include a harness assembly for manually manipulating the position and orientation of the platform and a foot support assembly to engage with the heal of the foot to further support the patient's foot and/or leg on the platform.

## Description

### FIELD OF INVENTION

This disclosure relates generally to an apparatus and method for obtaining measurements of a human appendage, and more particularly, to an apparatus and method for obtaining measurements of the contours of a human appendage held in a preferred physical configuration, for use in the formation of a digital model and subsequent fabrication of orthotic and prosthetic devices.

### BACKGROUND

Obtaining accurate tri-planer or three-dimensional measurements of a human appendage such as a leg, ankle, and/or foot, and more particularly an accurate determination of an appendage's shape and contours, is desirable for many purposes. Such determinations of shape and contours have commercial application such as, for example, for use in fabricating specialized and customized orthotic and prosthetic devices for use by individuals with disabilities or other physical challenges or limitations related to their appendages. The purpose of such orthotic and prosthetic devices is to improve and optimize bio-mechanical functions of the appendage and/or to correct functional problems that result from deficiencies in muscle function, bone structure, and/or associated soft tissues of the appendage. The shape, contours, and characteristics of each individual's appendages naturally vary from person to person; thus, the fabrication of applicable orthotic or prosthetic devices generally requires customized and specialized fabrication to match the individual's physical characteristics and best address the individual's needs and requirement. To fabricate such a customized and specialized orthotic or prosthetic device, it is essential to accurately determinate the shape and contours of the applicable human appendage in a tri-planer or three-dimensional orientation.

Traditional techniques for obtaining measurements of a patient's appendage, such as a lower leg, have been accomplished by taking a direct mold of the patient's foot, ankle, and any other required portion of the lower leg. Such a method requires that the foot be covered with plaster, silicone, resin, or similar molding material that hardens to the contours of the lower leg. The resulting mold is then filled with plaster or similar material to form an accurate representation of the lower leg. This representation is then used as a model to accurately fabricate the custom orthotic or prosthetic device. Such a method is labor intensive, time consuming, messy, and inconvenient for the patient and the medical professional alike. The patient must hold his or her foot or other appendage absolutely still to allow the molding material to be applied and then for the material to sufficiently dry and harden to form a useful representation of the foot or other appendage.

Alternatives to manual molding methods include digitally scanning an appendage to create a digital model representative of the shape and contours of the appendage. Such a digital model can be stored and subsequently transmitted to assist in fabricating an orthotic or prosthetic device for use with the appendage. Such a method may incorporate a camera or scanner that is rotated around the appendage to capture images about the entire appendage. The resulting digital model is then used to determine the required dimensions of a customized orthotic or prosthetic device. Such a system is able to produce a relatively accurate digital model that represents the contours and shape of the appendage and is generally faster, more accurate, and more efficient than traditional molding methods. An example of one such system is provided by Comb O&P, LLC, which includes a software application that allows a mobile device such as a smartphone to be used as an appendage scanning device for capturing three dimensional data to assist in creating the digital model and subsequently fabricating a custom orthotic or prosthetic device for such digital model.

However, the proper collection of three-dimensional data requires the patient to extend his or her appendage into free space and hold the appendage motionless for a sufficient period of time to allow the scanning device to be rotated about the appendage to fully capture all necessary three-dimensional data required to create a complete and accurate digital model of the appendage. As will be appreciated, in addition to being uncomfortable for the patient, it may be a difficult or impossible task for a patient that has limited strength or endurance regarding the applicable appendage. After all, the patient is having the appendage scanned because the patient has medical issues with that appendage. Therefore, such a method can result in errors during the gathering of three-dimensional data, which leads to inaccurate digital models, trial and error experimentation, and typically requires additional scans to successfully fabricate useful orthotics and prosthetics. As such, appendage fixture devices have been developed to assist with holding a patient's appendage in the correct position during the scanning process.

Conventional appendage fixture devices are known such as, for example, U.S. Patent No. 8,567,081 to Smith, which discloses an apparatus for determining contours of a patient's foot. In this patent the appendage must be placed in a neutral position within a rather large device to allow the scanner or imaging device to properly capture three-dimensional data that accurately represents the contours of the foot. Specifically, the device includes a support member that engages the foot only beneath a lateral forefoot area. The device then moves the foot relative to the support member to reactively load the foot in a rearward direction to lock the metatarsal joint of the foot to prepare the foot for scanning. However, such conventional appendage fixtures have limitations and inefficiencies. For example, such devices are typically very large and bulky and do not provide for dynamically adjusting the patient's appendage to best capture three-dimensional data. In fact, such devices are designed to hold the foot in a specific predetermined orientation during scanning. Additionally, such devices lack tri-planer adjustability for a patient's appendage; thus, the patient's appendance cannot be positioned with multiple degrees of freedom for simultaneous rotation in the coronal, axial, and sagittal planes. Contrary to the prior art devices and systems, each patient's appendage us unique and requires positioning of the appendage that is specific to that patient.

As such, it is desirable to provide an appendage fixture assembly that overcomes the deficiencies of the prior art that can sufficiently positioned a patient's appendage to capture three-dimensional data of the appendage when placed in the proper and customizable position to accurately construct a digital model that precisely and completely represents a patient's appendage. Such a digital model can subsequently be used to fabricate a proper customized orthotic or prosthetic device.

Accordingly, it is an objective of the present disclosure to provide an efficient and effective apparatus, system, and method of use for properly and customizably positioning the patient's appendage for the capturing of three-dimensional data and in particular for capturing a digital model for assisting in the fabrication of customized orthotic and prosthetic devices. No existing apparatus allows a patient's appendage, in particular a patient's lower leg, to be positioned dynamically so that an accurate digital model can be captured. These and other desirable characteristics of the disclosure will become apparent in light of the present specification, including claims, and figures.

### SUMMARY

The present disclosure describes and details a fixture assembly for dynamically positioning a human appendage in preparation of digital scanning of such appendage. The fixture assembly is arranged to provide for movement of the appendage in three-dimensions, including the ability to simultaneously rotate in the coronal, axial, and sagittal planes, while providing space for a scanning device to be rotated about the appendage to capture three-dimensional digital data.

In one embodiment an adjustable fixture assembly for supporting an appendage such as a foot and/or leg of a patient in a desirable position to allow for a comprehensive scan of the foot and/or leg by a scanner device. The adjustable fixture assembly comprising a base, a support member, and a platform. A ball joint secures a first end of the support member to the based and a second end of the support member is coupled to the platform. The adjustable fixture assembly is configured to support a foot and/or leg of a patient on the platform and the platform is configured to pivot about three degrees of freedom relative to the base. The adjustable fixture assembly can optionally include a harness assembly for manually manipulating the position and orientation of the platform. The adjustable fixture assembly can optionally include a foot support assembly to engage with the heal of the foot to further supporting the patient's foot on the platform.

### BRIEF DESCRIPTION OF THE DRAWINGS

In the accompanying drawings, structures are illustrated that, together with the detailed description provided below, describe example embodiments of the disclosed systems, methods, and apparatus. Where appropriate, like elements are identified with the same or similar reference numerals. Elements shown as a single component can be replaced with multiple components. Elements shown as multiple components can be replaced with a single component. The drawings may not be to scale. The proportion of certain elements may be exaggerated for the purpose of illustration.
FIG. 1 is a perspective view of an embodiment of the fixture assembly of the present disclosure.
FIG. 2 is a side view of the fixture assembly of FIG. 1.
FIG. 3 is a side view of the fixture assembly of FIG. 1 configured in a storage position.
FIG. 4 is another side view of the fixture assembly of FIG. 1.
FIG. 5 is yet another side view of the fixture assembly of FIG. 1.
FIG. 6 is a topside view of the fixture assembly of FIG. 1.
FIG. 7 is another perspective view of the fixture assembly of FIG. 1.
FIG. 8 schematically illustrates a patient placing a foot in a neutral position with assistance of an embodiment of a fixture assembly of the present disclosure.
FIG. 9 schematically illustrates the fixture assembly of the present disclosure with a patient's foot being scanned with a scanning device.
FIG. 10 schematically illustrates an embodiment of the fixture assembly of FIG. 8 with the patient's foot in a plantar flexion position.
FIG. 11 schematically illustrates an embodiment of the fixture assembly of FIG. 8 with the patient's foot in a dorsiflexion position.
FIG. 12 schematically illustrates an embodiment of the fixture assembly of FIG. 8 with the patient's foot in a supination position.
FIG. 13 schematically illustrates an embodiment of the fixture assembly of FIG. 8 with the patient's foot in a pronation position.
FIG. 14 schematically illustrates an embodiment of the fixture assembly of FIG. 8 with the patient's foot in an inwardly rotated position.
FIG. 15 schematically illustrates an embodiment of the fixture assembly of FIG. 8 with the patient's foot in an outwardly rotated position.
FIG. 16 is a perspective view of another embodiment of the fixture assembly of the present disclosure.
FIG. 17 is a perspective view of yet another embodiment of a fixture assembly of the present disclosure.
FIG. 18 is a top view of the fixture assembly of FIG. 17.
FIG. 19 is a side view of the fixture assembly of FIG. 17.
FIG. 20 is a front view of the fixture assembly of FIG. 17.
FIG. 21 is another side view of the fixture assembly of FIG. 17.
FIG. 22 is a side view of the fixture assembly of FIG. 17 with the foot holder in a first position.
FIG. 23 is a side view of the fixture assembly of FIG. 17 with the foot holder in a second position.
FIG. 24 is a side view of the fixture assembly of FIG. 17 arranged in the dorsiflexion position.
FIG. 25 is a side view of the fixture assembly of FIG. 17 arranged in the plantar flexion position.
FIG. 26 is a side view of the fixture assembly of FIG. 17 arranged in an inward/outward position (depending on whether it is the patient's left or right foot on the platform).
FIG. 27 is a side view of the fixture assembly of FIG. 17 arranged in an inward/outward position (depending on whether it is the patient's left or right foot on the platform).
FIG. 28 is a side view of the fixture assembly of FIG. 17 arranged in the in the pronation/supination position (depending on whether it is the patient's left or right foot on the platform).
FIG. 29 is a side view of the fixture assembly of FIG. 17 arranged in the arranged in the pronation/supination position (depending on whether it is the patient's left or right foot on the platform).
FIG. 30 illustrates several views of the fixture assembly of FIG. 17 in a compact or collapsed position.

### DETAILED DESCRIPTION

Reference will now be made in detail to embodiments of the present teachings, examples of which are illustrated in the accompanying drawings. It is to be understood that other embodiments may be utilized and structural and functional changes may be made without departing from the scope of the present teachings. Moreover, features of the embodiments may be combined, switched, or altered without departing from the scope of the present teachings, e.g., features of each disclosed embodiment may be combined, switched, or replaced with features of the other disclosed embodiments. As such, the following description is presented by way of illustration and does not limit the various alternatives and modifications that may be made to the illustrated embodiments and still be within the spirit and scope of the present teachings.

As used herein, the words "example" and "exemplary" mean an instance, or illustration. The words "example" or "exemplary" do not indicate a key or preferred aspect or embodiment. The word "or" is intended to be inclusive rather an exclusive, unless context suggests otherwise. As an example, the phrase "A employs B or C," includes any inclusive permutation (e.g., A employs B; A employs C; or A employs both B and C). As another matter, the articles "a" and "an" are generally intended to mean "one or more" unless context suggests otherwise.

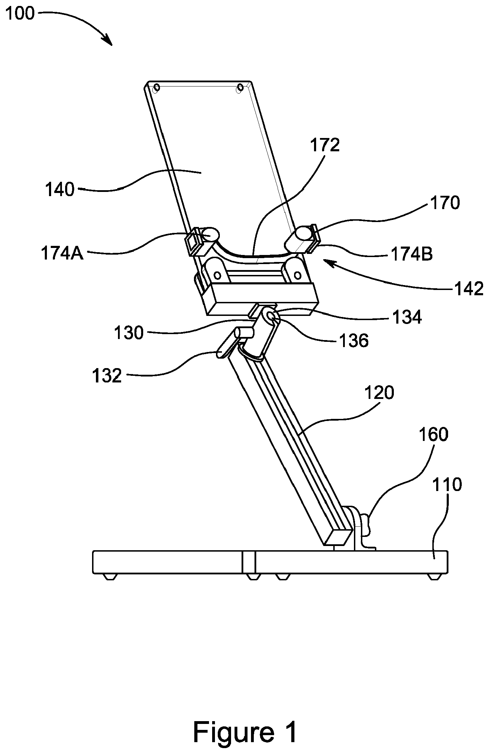

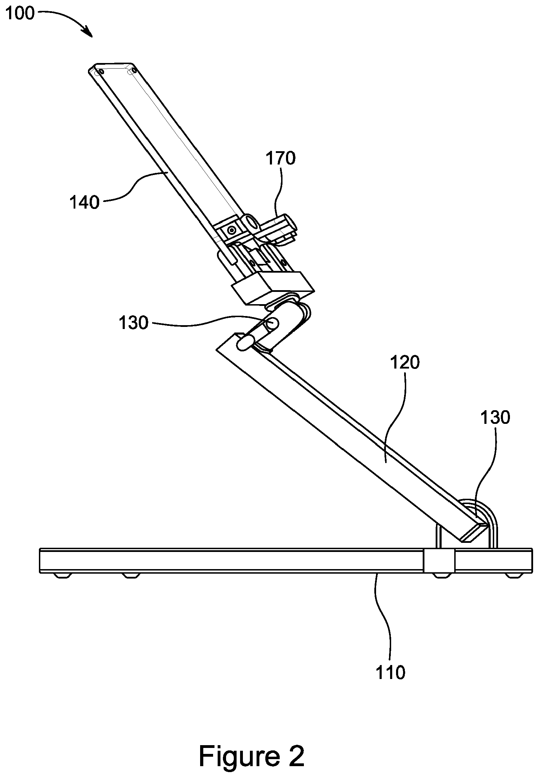

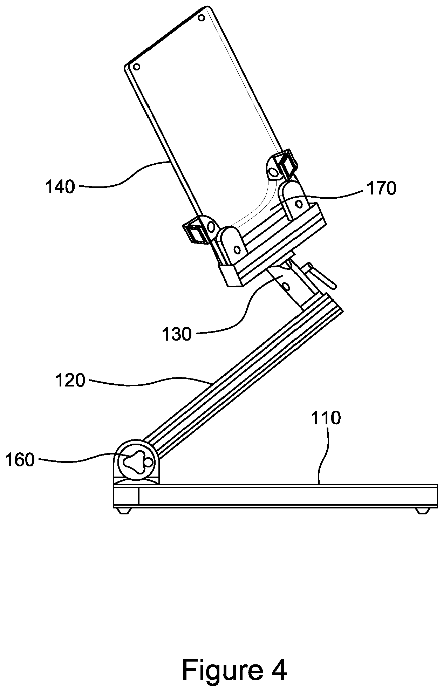

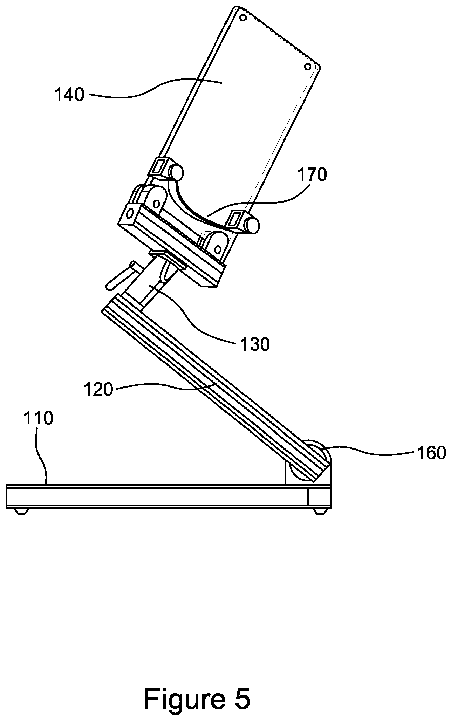

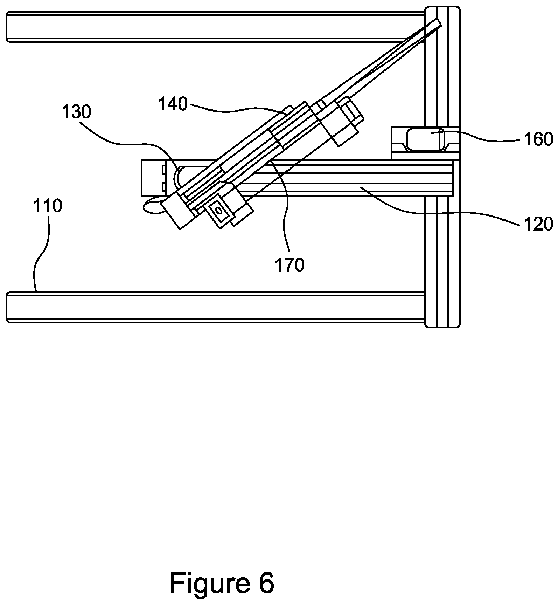

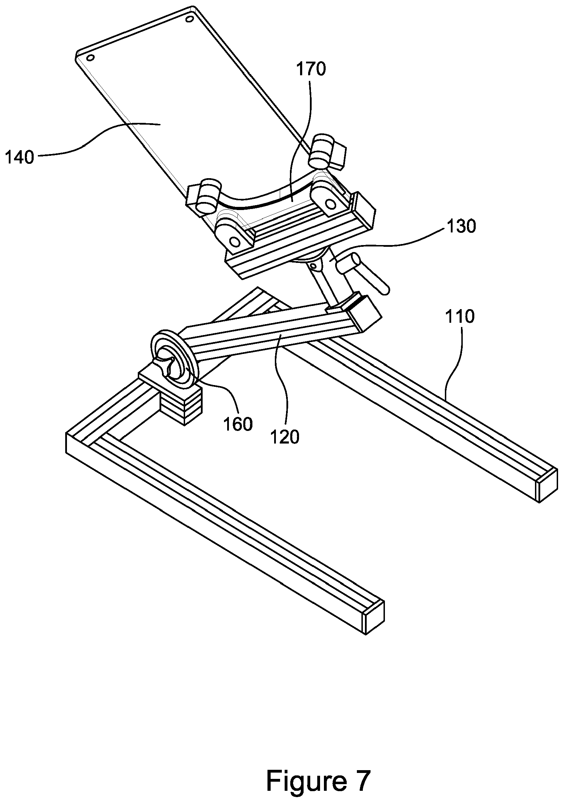

FIGS. 1-15 illustrate one embodiment of a fixture assembly 100. The fixture assembly 100 is arranged to provide support for a patient to place and maintain his or her foot and/or leg in a specific and desired location and orientation. The fixture assembly 100 provides for dynamically adjusting the location of the foot and/or leg into a plurality of positions and orientations as needed, so that the foot and any related bodily structures (such as the patient's ankle or leg) can be inspected and examined by a clinician and subsequently can be properly and efficiently scanned with a handheld or automated device. The fixture assembly 100 is configured to allow the foot, ankle, and leg to be dynamically positioned and oriented by the patient and/or clinician, with the ability to simultaneously move and rotate the foot about all three degrees of freedom, as required.

As illustrated in the figures, the fixture assembly 100 includes a base 110 with a support member 120 that extends upward from the base 110 toward a platform 140. The platform 140 is generally arranged so that a patient can rest his or her foot on the platform 140. The platform 140 has a generally flat structure and is transparent so that a scanning device can scan through the platform 140. In this example, the platform 140 is generally rectangular in shape; however, as will be subsequently described, the platform can be other shapes as well. In the embodiment illustrated in FIGS. 1-15, a locking pivot holder 130 is attached to the end of the support member 120 opposing the base 110 and is arranged to couple the platform 140 to that end of the support member 120. The locking pivot holder 130 is arranged to accommodate the rotational movement of the platform 140 through all three (3) degrees of freedom ("DOF") so that the patient's foot (and correspondingly, the patient's leg) can be positioned in a beneficial location and orientation to facilitate examination and scanning.

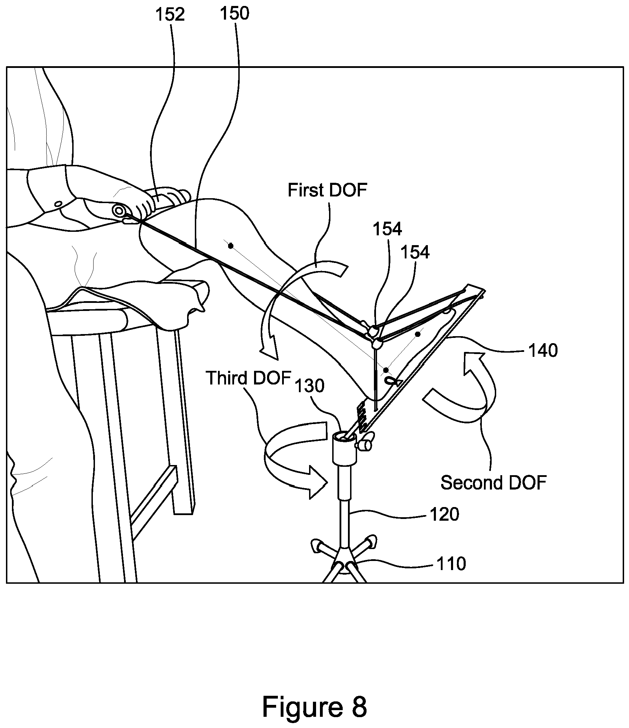

As illustrated in FIGS. 8-15, a string harness 150 with a handle 152 and adjustable members 154 can be coupled to the platform 140. In one example, the string harness 150 is coupled proximate to each of the four corners of the rectangular shaped platform 140. As will be subsequently described, the string harness 150 and adjustable members 154 can be manually manipulated and adjusted by the patient or the clinician to position the patient's foot (and correspondingly, the patient's leg) in a desirable location and orientation to facilitate examination and scanning.

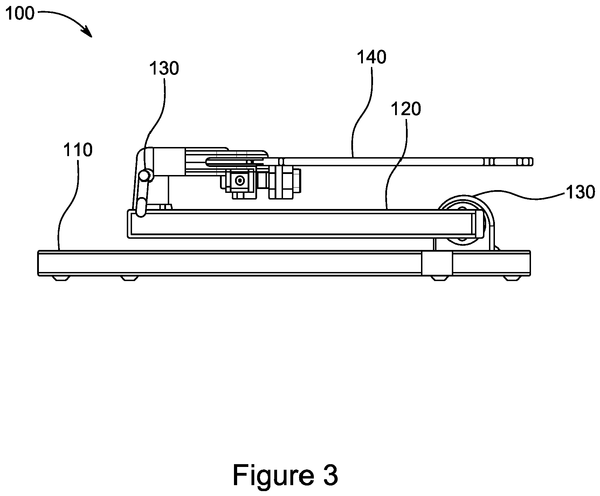

The base 110 and support member 120 can be coupled together by a base holder 160. The base holder 160 can be arranged to allow the support member 120 to pivot relative to the base 110 and to lock the support member 120 in either a compact position (as illustrated in FIG. 3) or in a use position (as illustrated in FIG. 1). A foot holder 170 may be positioned on the platform 140 to support a patient's foot relative to the platform 140 through interaction with the patient's heal or the back of the patient's foot. In the embodiment illustrated, the foot holder 170 includes a flexible strap 172 and a pair of pegs 174A, 174B attached to opposite ends of the flexible strap 172 to secure the flexible strap 172 to the platform 140. Although the foot holder 170 includes a flexible strap 172, any type of foot holder 170 is contemplated to be used herein to allow for a patient's foot to be positioned and supported relative to the platform 140. In one example, a platform base 142 may extend from the platform 140 to allow for pivotal movement and locking attachment of the platform 140 to the locking pivot holder 130.

The locking pivot holder 130 may include a handle 132 configured to be actuated to place the locking pivot holder 130 in either a locked or unlocked position. The locking pivot holder 130 may include a ball joint 136 that extends through a slot 134 to attach to the platform base 142. The slot 134 may allow the ball joint 136 positioned within the locking pivot holder 130 to be pivotally adjustable in all 3 DOF as illustrated by FIGS. 8-15. Further, the holder 132 may be positioned along the support member 120, where such positioning can be along any point of the length of the support member 120.

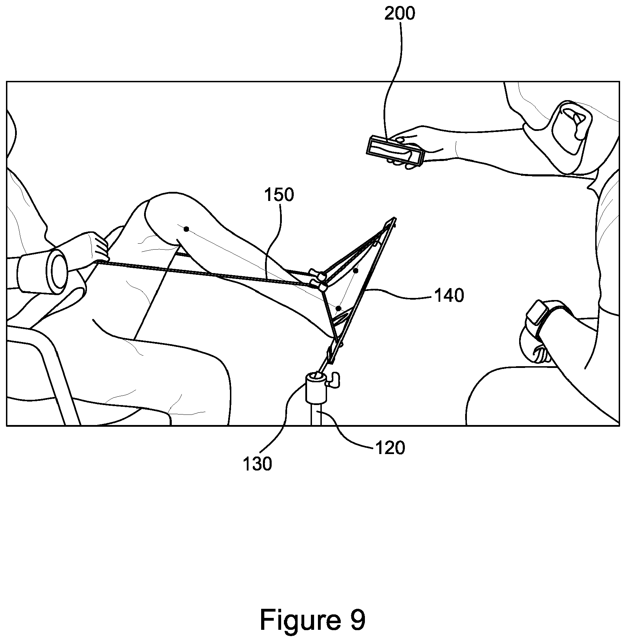

The fixture assembly 100 as described herein may find particular use as a device that supports a patient's foot during the digital scanning the patient's foot and/or leg. The fixture assembly 100 is arranged so that a patient can place his or her foot on the platform 140, resting his or her heal in the foot holder 170, and using the string harness 150 to manipulate the position of the foot (and correspondingly, the position of the leg) until it is in the desired location and orientation. Once in the desired location and orientation, a clinician can move a scanning device 200 (usually a camera on a smartphone as illustrated by FIG. 9) 360 degrees about the foot and/or leg to capture a comprehensive physical scan of the foot and associated areas such as the ankle and leg. As will be appreciated, when the position of the foot is being manipulated and adjusted, the locking pivot holder 130 is in an unlocked position. Once the foot ((and correspondingly, the leg) is positioned in the desired location and orientation, the handle 132 can be actuated to secure its ball joint 136 in place within the slot 134, which statically secures the position of the locking pivot holder 130, which in turn, statically secures the position of the platform 140. Thus, the patient can rest comfortably with his or her foot statically positioned on the platform 140 in a desired location and orientation as the technician scans the patient's foot, ankle, and/or leg.

When the patient places his or her foot onto the platform 140, all portions of the bottom of the patient's foot are generally in contact with the platform 140. As will be described herein with reference to FIGS. 8-15, such significant contact between the bottom of the patient's foot and the platform 140 results in the foot generally moving in the direction that the platform 140 moves. Such coordinated movement is due to the friction between the bottom of the patient's foot and the platform 140. Additionally the foot holder's 170 engagement with the heal or back of the foot can assist in such coordinated movement.

As illustrated by FIG. 8, the platform 140 is configured to be rotationally adjustable about all 3 rotational degrees of freedom. Additionally, the support member 120 can be adjusted relative to the base 110 to further adjust the height and lateral position of platform 140. FIG. 9 illustrates the fixture assembly 100 as it is in use with a scanning device 200 such as a cell phone or camera that is capable of being moved 360 degrees about the appendage of the patient to produce a comprehensive scan of the shape and contours of the patient's foot, ankle, and/or leg. Notably, in FIG. 9 a laser alignment marker has been included to identify a desirable pivotal position of the foot relative to the ankle of the patient to capture the desired images and digital data with the scanning device 200. Notably, it is desirable to be able to procure these comprehensive scans of the patient as the foot, ankle, and/or leg is placed in various positions, in the three separate adjustable planes, which can be accomplished by the adjustable fixture assembly 100 of the present disclosure.

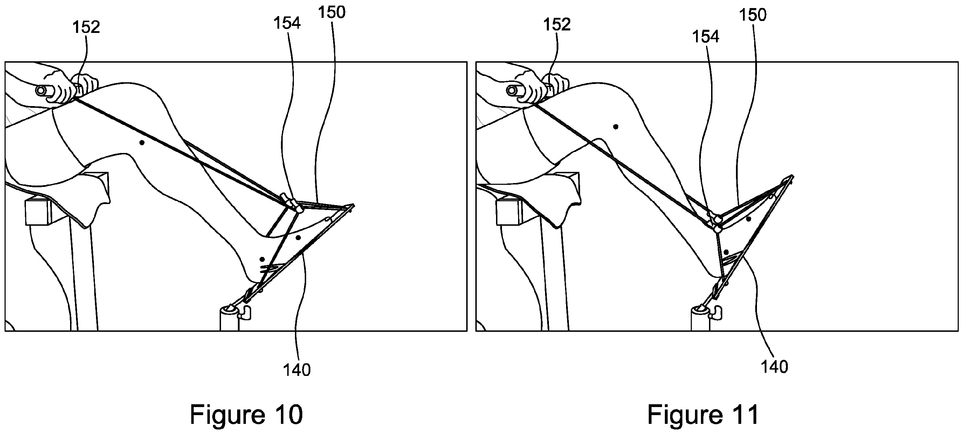

As noted, rotational movement of the platform 140 may be accomplished while a patient's foot is resting on the platform 140. The first DOF (as illustrated and labeled in FIG. 8) is defined as between dorsi and plantar positions. Rotation in this first DOF may be accomplished by use of the string harness 150 controlled by the patient or clinician to position the platform 140 along the axis of rotation about the patient's ankle to allow for such dorsi-plantar movement. Such movement is illustrated in FIGS. 10 and 11. The user can extend the handle 152 to allow for the platform 140 to pivot towards a plantar flexion position as illustrated by FIG. 10. In this position, the adjustable members 154 may be adjusted along the string hardness along both sides of the assembly and positioned or moved towards a top of the platform 140. Also, the user can pull the handle 152 to allow the platform to pivot towards a dorsiflexion position as illustrated by FIG. 11. In this position, the adjustable members 154 may be moved along the string harness 150 to positions toward a bottom of the platform 140.

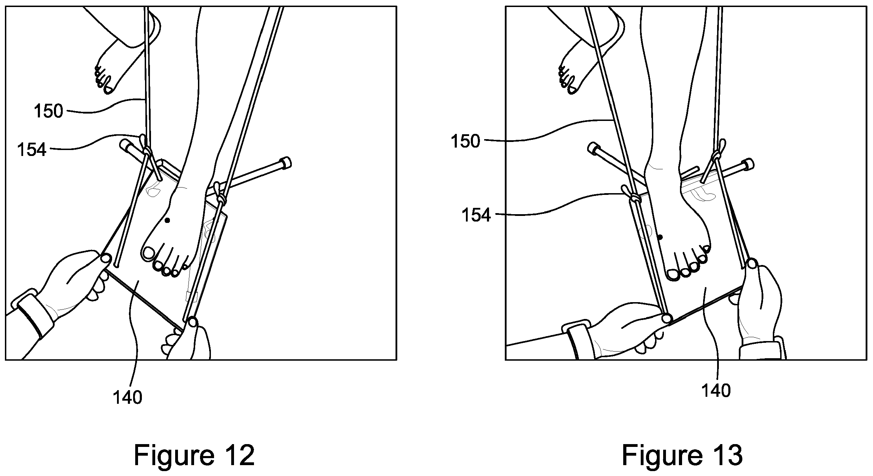

The second DOF (as illustrated and labeled in FIG. 8) is defined as between supination and pronation positions of a patient's ankle and foot. Such movement is illustrated in FIGS. 12 and 13. The platform 140 can be pivoted inwardly to locate the patient's foot in a supination position as illustrated by FIG. 12. Also, the platform 140 can be pivoted outwardly to locate the patient's foot in a pronation position as illustrated by FIG. 13. As previously noted, friction between the bottom of the patient's foot and the platform 140 results in the foot pivoting inward or outward. In the second DOF, the platform 140 is rotatable along a longitudinal axis of the platform 140 to allow for pronation-supination movement, with such movement facilitated by the patient or with assistance from a clinician.

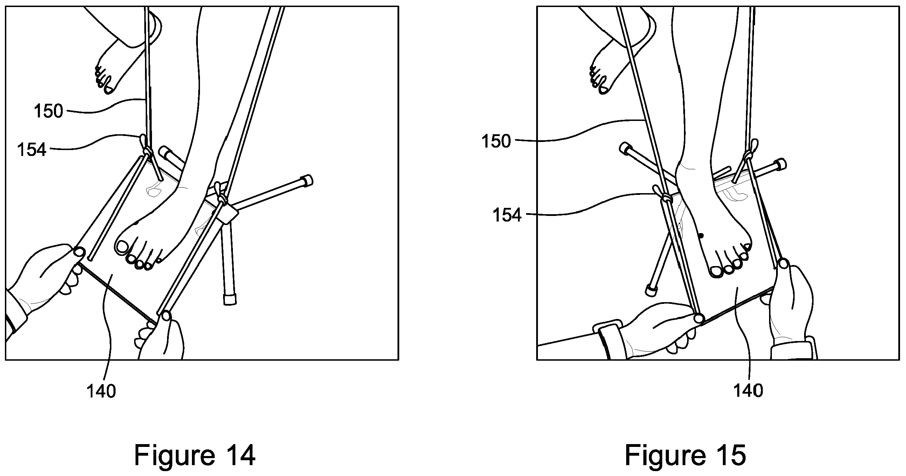

The third DOF (as illustrated and labeled in FIG. 8) is defined as between an inwardly rotated position and an outwardly rotated position of a patient's ankle and foot along the plane of the bottom of the foot. Such movement is illustrated in FIGS. 14 and 15. The platform 140 can be pivoted inwardly to locate the patient's foot in an inwardly rotated position as illustrated by FIGS. 14. Also, the platform 140 can be pivoted outwardly to locate the patient's foot in an outwardly rotated position as illustrated by FIG. 15. In the third DOF, the platform 140 is rotatable along an axis of the support member 120 rotate the position of the foot relative to the leg, with such movement facilitated by the patient or with assistance from a clinician.

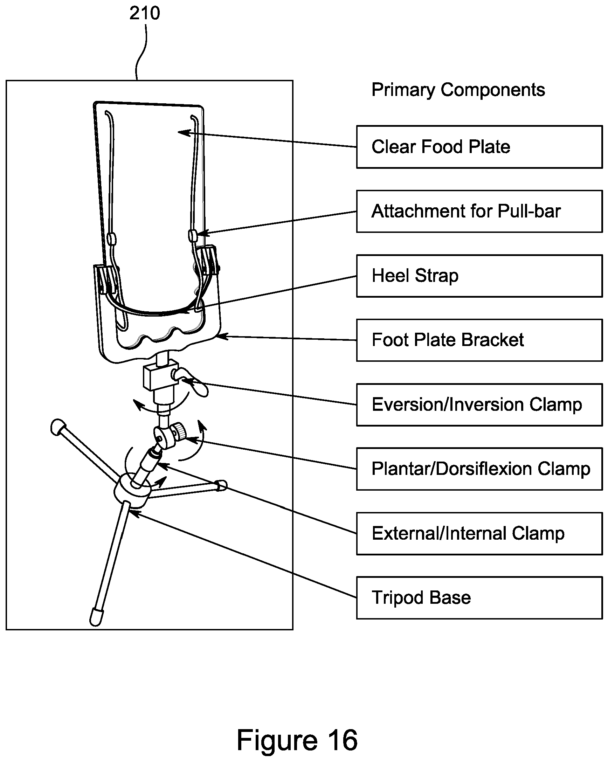

FIG. 16 illustrates another embodiment of the fixture assembly 210 which is described to include various components having similar configurations and functions as the components described in FIGS. 1-15. Here the fixture assembly 210 includes a clear platform (foot plate), an attachment for pull-bar (string harness), a heel strap, a foot plate bracket, and a base. However, instead of using a locking pivot holder, the fixture 100 may include a plurality of clamps such as an eversion/inversion clamp, a plantar/dorsiflexion clamp, and an external/internal clamp as illustrated by FIG. 16. These clamps may separately allow the clear foot plate to pivot about the three degrees of freedom as described above. Notably, the base is illustrated as a tripod base which may allow the fixture assembly 210 to be placed into a compact or storage position when not in use.

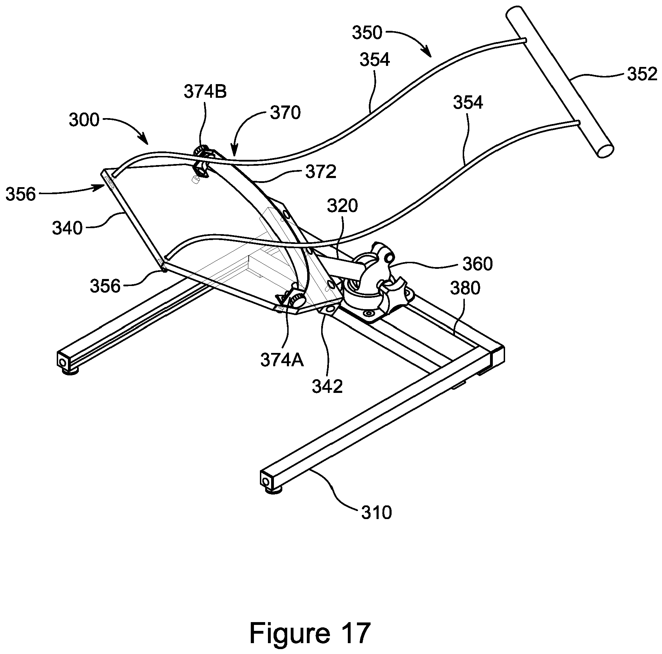

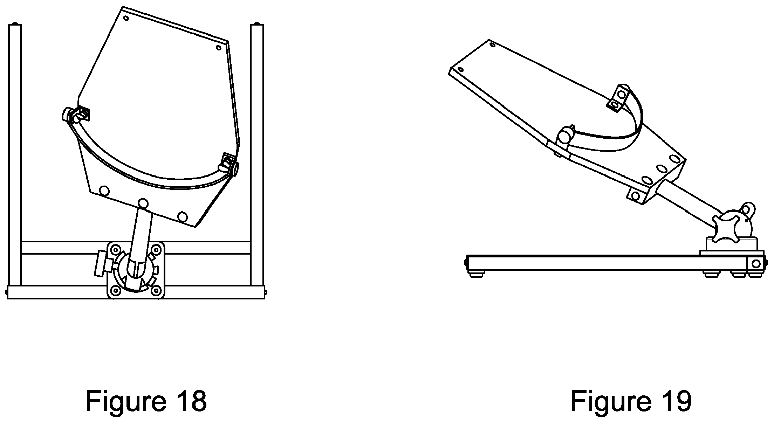

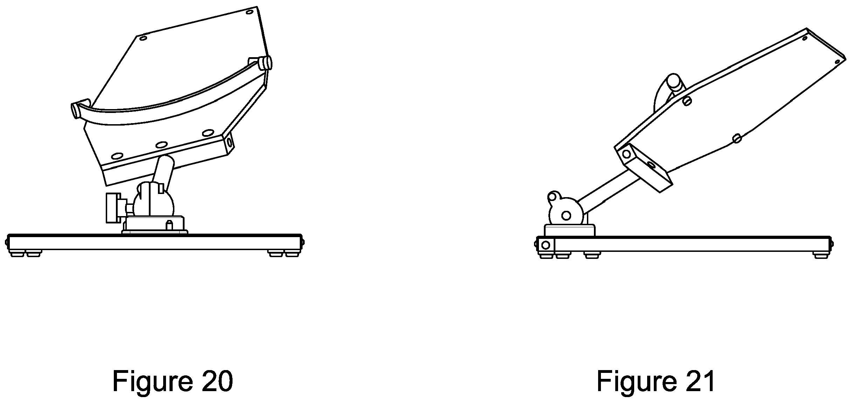

Another embodiment of the fixture assembly 300 is illustrated in FIGS. 17-30. This fixture assembly 300 shares a number of similarities with the other fixture assemblies 100, 210 described herein, and includes a number of notable differences. The fixture assembly 300 includes a base 310, a support member 320, a platform 340, and a ball joint 360 coupling a first end of the support member 320 to the base 310. In the embodiment illustrated, a second end of the support member 320 is statically secured to the platform 340 by a series of fasteners. In other embodiments, the second end of the support member 320 can be coupled to the platform 340 with an adjustable and dynamic coupling that can provide for movement of the platform 340 relative to the support member 320. A harness assembly 350 is secured to the platform 340 to facilitate manual manipulation of the three-dimensional orientation of the platform 340 to arrange the platform 340 in its desired position for a scan of an appendage. The fixture assembly 300 includes a foot support assembly 370 positioned near the bottom edge of the platform 340 to support a patient's foot on the platform 340.

The platform 340 is a generally flat structure fabricated from transparent material; however, unlike prior embodiments, the platform 340 is trapezoid shaped. Such a shape accommodates both adult and pediatric uses. The material used to fabricate the platform 340 is abrasion and impact resistant and is further resistant to ultraviolet light. The support member 320 is tubular in structure and is attached to the base 310 with the ball joint 360. The ball joint 360 provides for greater rotational movement of the support member 320 (and thus, the platform 340) relative to the base 310. The ball joint 360 includes a mechanism for locking the ball joint 360 in a given position. When a patient places his or her foot on the platform 340, the locking mechanism of the ball joint 360 can be loosened to provide for free movement of the support member 320 and platform 340. The patient and/or a clinician can manipulate the orientation of the platform 340 in all three planes (i.e., the plantar/dorsiflexion plane, eversion/inversion plane, and adduction/abduction plane) to achieve the desired position. Once the desired position is achieved, the locking mechanism of the ball joint 360 can be tightened to secure the position of the ball joint 360, which correspondingly secures the position of the platform 340. The locking mechanism of the ball joint 360 can be arranged so that it can be loosened but the ball joint 360 continues to provide modest resistance to movement. Such a configuration may make it easier for a patient or clinician to finely adjust the precise position and orientation of the platform 340. As will be further discussed, the ball joint 360 further facilitate collapsing the fixture assembly 300 into a compact arrangement for efficient storage of the fixture assembly 300.

The harness assembly 350 is arranged to optionally assist a patient or clinician in manipulating the position of the platform 340. The harness assembly 350 includes a handle 352 and two cables 354 extending from the handle 352. The two cables 354 are secured to the top two corners of the platform 340 with a pair of securing mechanisms 356. The securing mechanisms 356 can be arranged to reversibly attach the harness assembly 350 to the platform 340 so that the patient or clinician can optionally remove the string harness 350 from the platform 340 if it is no longer needed or desired. In one example, the securing mechanisms 354 are T-joints that can be inserted into holes in the platform 340 and rotated to secure the harness assembly 350 to the platform 340. The T-joints 354 can be optionally counter-rotated and removed through said holes to disengage the harness assembly 350 from the platform 340. The length of the cables 354 are adjustable via the handle 352 to accommodate the height and size of each patient. In one example, the length of the cables 354 can be adjusted by the patient or clinician rolling or coiling the cables 354 around the handle 352. It will be understood that once the ball joint 360 is loosened, the harness assembly 350 can be optionally used to manually manipulate the position and orientation of the platform 340 to achieve the desired precise position and orientation for the foot (and correspondingly, the leg) for subsequent scanning of the foot, ankle, and/or leg of the patient. Once such a desired position and orientation is achieved, the ball joint 360 can be tightened and the harness assembly can be released or removed from the platform 340. It will be understood that the platform 340 can be manipulated by the patient or clinician to achieve a desired orientation and position without the use of the harness assembly 350.

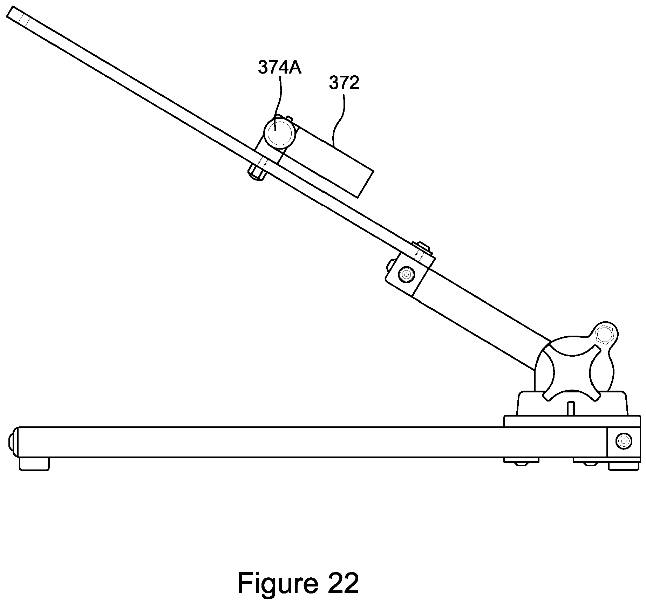

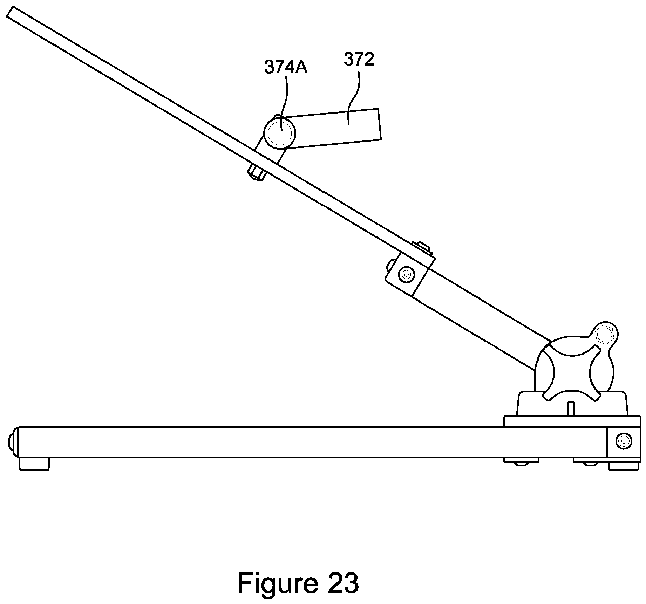

The foot support assembly 370 is arranged so that it is adjustable to accommodate the shape and size of the patient's foot, including both adult and pediatric patients. The foot support assembly 370 includes a heal strap 372 and a pair of rotating knobs 374A, 374B that can be tightened and loosened as required to adjust the rotational position of the heal strap 372. Once the rotating knobs 374A, 374B are loosened, the heal strap 372 can be rotated about the rotating knobs 374A, 374B (as illustrated in FIGS. 22 and 23) between all positions along a 180 degree path to accommodate individual patients. Once the heal strap 372 is located in a desirable position, the rotating knobs 374A, 374B are tightened to maintain the heal strap 372 at this desirable position. In another embodiment, the foot support assembly 370 includes a heal strap and a pair of rotatable couplings that rotatably secure the heal strap to the platform. In essence, the rotatable couplings allow the heal strap to freely rotate along the 180 degree path. The heal strap can be fabricated from transparent material as not to interfere with the scanning of the foot, ankle, or leg. The fixture assembly further includes a branding plate 380 to further support the base 310 and provide for an aesthetically pleasing appearance.

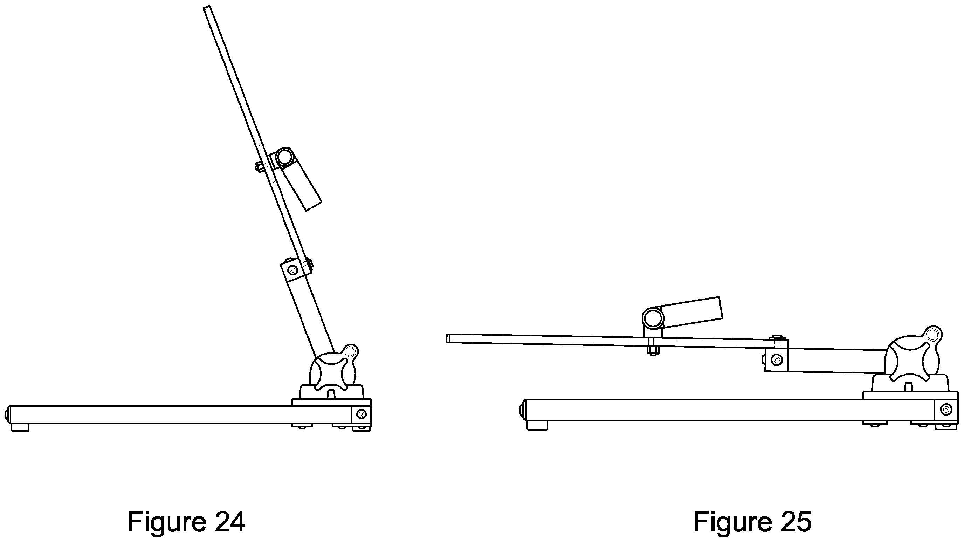

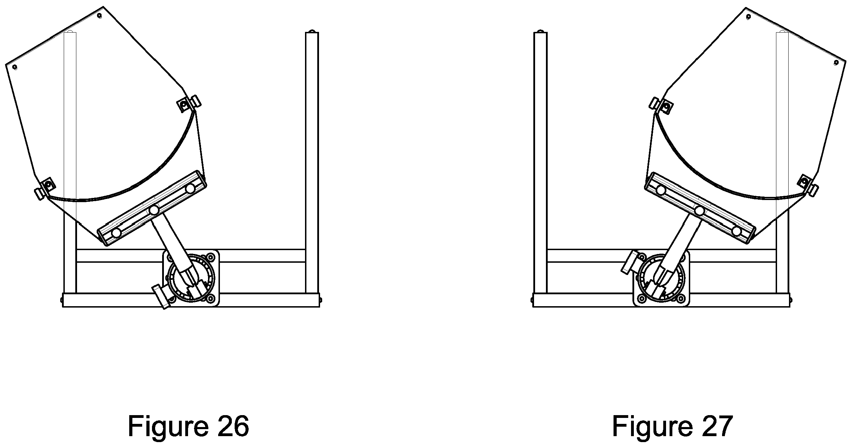

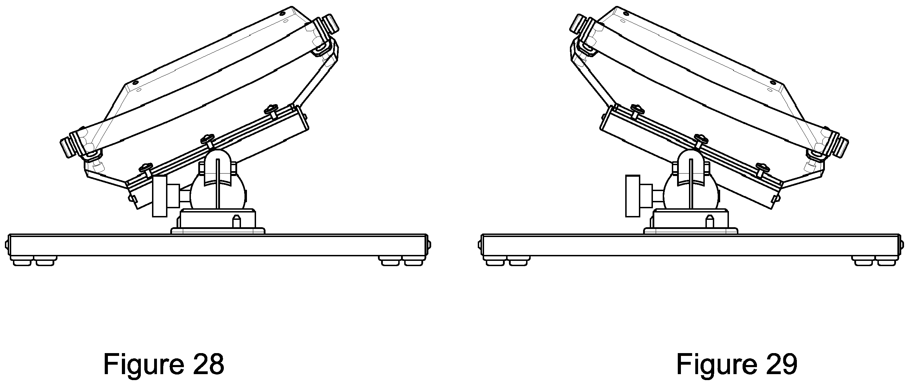

FIGS. 24-29 illustrate an example of the rotational range of the fixture assembly 300. FIGS. 24 and 25 illustrate the plantar-dorsiflexion rotation of the fixture assembly 300. FIGS. 26 and 27 illustrate the adduction-abduction rotation of the fixture assembly 300. FIGS. 28 and 29 illustrate the eversion-inversion rotation of the fixture assembly 300.

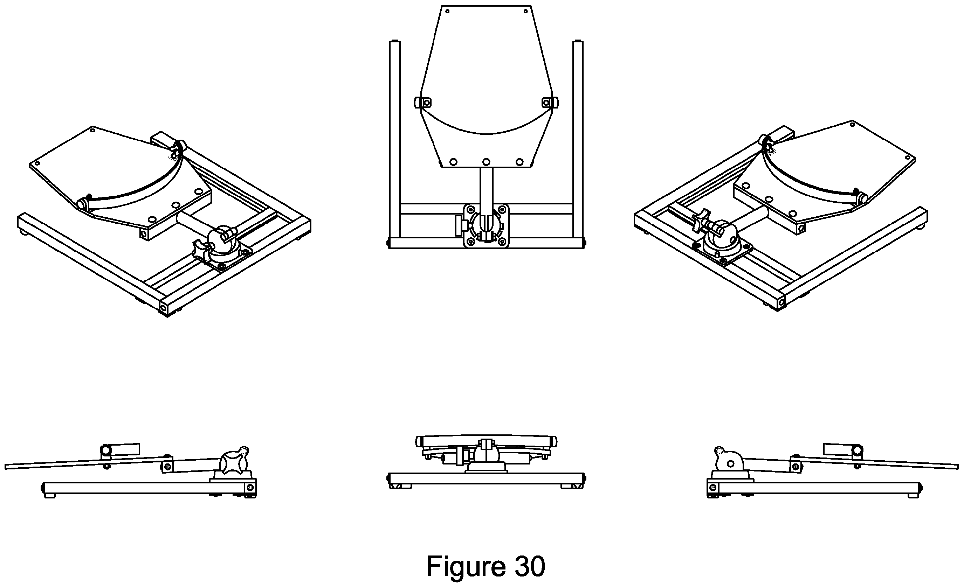

As noted above, the fixture assembly 300 can be positioned in a compact or collapsed position for storage or transportation. FIG. 30 illustrates a number of views of the fixture assembly 300 in such a compact or collapsed position. In order to move the fixture assembly 300 from the operative position to the storage position, the ball joint 360 is loosened and the platform 340 and support member 320 are rotated downward so that the platform 340 is generally positioned proximate to and parallel to the base 310. Once in such a position, the ball joint 360 can be tightened to lock the fixture assembly 300 in the collapsed position. This process is then reversed to return the fixture assembly 300 to the operative position. It will be appreciated that when in the storage position, the fixture assembly 300 is easy to move, transport, and store. In one example, the fixture assembly 300 in the storage position can be placed in a carrying case for ease of transport and to protect the fixture assembly 300 during transport.

In another embodiment, a leg support assembly can be used with a fixture assembly to directly support the leg of a patient. The leg support assembly can be arranged to engage with the underside of a patient's knee to support the patient's leg during the scanning process. Such support results in the patient's leg and foot remaining still and stable during the scanning process, which correspondingly results in a more accurate and precise scan. In one example, the leg support assembly include a base, a height-adjustable member, and an under-knee support. The base can be arranged to engage with the floor or other stable surface to provide a solid foundation for the leg support assembly. The height-adjustable member can be a telescoping tube assembly or other such arrangement that allows a user, such as a patient or clinician, to adjust the length of the member. The height-adjustable member is attached on one end to the base and on the other end to the under-knee support. The under-knee support can be a cushioned member that is oriented generally perpendicular to the height-adjustable member. In one example, the under-knee support member can resemble the top portion of a crutch with a pad covering a slightly concave member that rests under the back of a patient's knee. As will be appreciated, the height of the leg support assembly can be adjusted, via the height-adjustable member, to accommodate patient of different heights (i.e., with shorter and longer legs). The leg support member can be generally positioned under the patient's knee at a desirable height, the patent can then place his or her foot on the platform, the patient or clinician can manually adjust the orientation and position of the platform to achieve the desired position and orientation of the foot, ankle, and/or leg, and the clinician can capture a scan of the patient's foot, ankle, and/or leg. It will be appreciated that in addition to adding stability to the patient's leg, a leg support assembly can also prevent the patient's leg from hyperextending during the manipulation of the foot via the platform.

Although the embodiments of the present disclosure have been illustrated in the accompanying drawings and described in the foregoing detailed description, it is to be understood that the present disclosure is not to be limited to just the embodiments disclosed, but that the disclosure described herein is capable of numerous rearrangements, modifications and substitutions without departing from the scope of the claims hereafter. The claims as follows are intended to include all modifications and alterations insofar as they come within the scope of the claims or the equivalent thereof.

## Claims

1. An adjustable fixture assembly comprising:
a base;
a support member;
a ball joint coupling a first end of the support member to the base;
a platform coupled to the second end of the support member, where the platform is fabricated from a transparent material and is configured to support a foot of a patient.

2. The adjustable fixture assembly of claim 1, wherein the platform is configured to pivot about all three degrees of freedom relative to the base.

3. The adjustable fixture assembly of claim 2, wherein ball joint facilitates the pivoting of the platform about all three degrees of freedom relative to the base.

4. The adjustable fixture assembly of any preceding claim, wherein the ball joint includes a mechanism for locking and unlocking the ball joint.

5. The adjustable fixture assembly of claim 2 or 3, wherein the three degrees of freedom include a first degree of freedom between a plantar flexion position and a dorsiflexion position, a second degree of freedom between a supination position and pronation position, and a third degree of freedom between an inwardly rotated position and an outwardly rotated position.

6. The adjustable fixture of any preceding claim, wherein the platform is statically coupled to the second end of the support member.

7. The adjustable fixture of any of claims 1 to 5, wherein the platform is coupled to the second end of the support member by a rotatable coupling member.

8. The adjustable fixture assembly of any preceding claim, further comprising a harness assembly with a handle and a pair of adjustable cables extending from the handle and securable to the platform.

9. The adjustable fixture assembly of claim 8 wherein pair of adjustable cables are securable to the platform by a pair of Tjoints.

10. The adjustable fixture of any preceding claim, further comprising a foot support assembly.

11. The adjustable fixture of claim 10, wherein the foot support assembly includes a heal strap and a pair of rotating knobs that secure the heal strap to the platform.

12. The adjustable fixture of claim 11, wherein the heal strap is secured to the platform proximate to a bottom edge of the platform.

13. The adjustable fixture of claim 11 or 12, wherein the heal strap is rotatably adjustable by loosening the rotating knobs.

14. The adjustable fixture of claim 10, wherein the foot support assembly includes a heal strap and a pair of rotatable couplings that secure the heal strap to the platform.

15. The adjustable fixture of claim 14, wherein the heal strap is secured to the platform proximate to a bottom edge of the platform and/or wherein the heal strap can freely rotate about the rotatable couplings.
